# EUROPEAN PATENT SPECIFICATION

(11) **EP 2 544 603 B1**
(45) Date of publication and mention of the grant of the patent: **10.05.2017**
(21) Application number: 11709288.2
(22) Date of filing: 10.03.2011
(51) Int. Cl.: A61B 17/221

(54) **OBSTRUCTION REMOVAL ASSEMBLY**
ANORDNUNG ZUR BESEITIGUNG VON OBSTRUKTIONEN
ENSEMBLE D'ÉLIMINATION D'OBSTRUCTION

(30) Priority: 12.03.2010 GB 201004126
(43) Date of publication of application: 16.01.2013
(73) Proprietor: Cook Medical Technologies LLC, Bloomington, IN 47404 (US)
(72) Inventor: AGGERHOLM, Steen, DK-4660 St. Heddinge (DK); CHRISTENSEN, Bente, Weber, DK-4100 Ringsted (DK); HENDRIKSEN, Per, DK-4160 Herlufmagle (DK); LYSGAARD, Thomas, DK-2680 Solroed Strand (DK); MOLGAARD-NIELSEN, Arne, DK-2100 Copenhagen (DK); SVENDSEN, Frank, DK-4100 Ringsted (DK)
(74) Representative: Jehan, Robert
(86) International application number: PCT/US2011/027894
(87) International publication number: WO 2011/112809

(56) References cited:
- WO-A1-98/39053
- WO-A2-02/094111
- US-A1- 2004 199 201
- US-A1- 2005 283 166

## Description

### Technical Field

The present invention relates to an obstruction removal assembly. The assembly may be used for the physical removal of thrombi from blood vessels.

### Background Art

Mechanical thrombectomy is a procedure that has been in widespread use for many years. Typical thrombectomy devices are balloons that are inflated in a vessel and then withdrawn to pull clots into a sheath which can be withdrawn from the patient to remove the clots. Other devices are simple open ended catheters into which a clot is aspirated and removed from the patient. Another thrombectomy device employs a basket device that is opened within the clot so that the clot becomes captured in the basket. The basket can then be retrieved along with the clot. Still other devices use a small corkscrew shaped device that is collapsed inside a catheter. The catheter is passed through the clot, the corkscrew is pushed out of the catheter allowing the device to expand, capturing the clot for removal. Some corkscrew devices are simply "screwed" into the clot, then retracted into a catheter for removal before the corkscrew is retracted.

US 6,511,492, US 6,669,721, US 6,692,509, US 7,316,692 and US 2005/0283166 disclose various designs of prior art clot removal devices.

WO 98/39053 A1, upon which the preamble of claim 1 is based, discloses an emboli capturing system comprising an expandable filter and an associated catheter wherein the filter has an expanded disposition with a tapered shape and having a chamber for capturing emboli and a withdrawal mechanism. WO 02/094111 A2 and US 2004/199201 A1 also disclose systems for removing obstructions from body lumens.

Aspirating the clot into a catheter can cause breaking up of the clot during the procedure. Furthermore, where a clot is located in a very narrow vessel, the size of the catheter is too small, and thus it is not practical to remove a clot by this method.

Corkscrew devices that are screwed into the clot usually have a smooth rounded tip to prevent the corkscrew from penetrating the vessel wall or otherwise damaging the vessel wall as it is screwed into the clot. With
these devices, however, the smooth, rounded central tip does not screw into the clot, but instead is pushed into the clot and then the remainder of the corkscrew is screwed into the clot. This results in a pushing force on the center of the clot and a pulling force on the periphery of the clot. These counter forces tend to macerate or fragment the clot and can result in only a small part of the clot being captured. Some corkscrew devices may substitute a sharp tip that can screw directly into the clot for the rounded tip. However, sharp tips can penetrate the vessel wall just as easily as they can penetrate and capture the clot. Such devices are seldom used since they carry the very high risk of penetrating the vessel wall. When a bead or ball is applied to the tip of the device that is large enough to protect the vessel wall, it will be so large that it will tend to push the clot distally rather than penetrate the clot such that the clot can be captured and removed.

Still another issue includes capturing any of the loose fragments possibly dislodged from the clot during removal of the clot. Another issue arises if a thrombectomy device has a diameter smaller than the vessel diameter when deployed from the catheter. In such a case, some of the clot immediately adjacent the vessel wall may not be removed.

Another issue associated with conventional thrombectomy devices is that they are typically too large and too stiff for use in the small tortuous vessels in the brain. Some of the conventional devices also use a central mandrel wire or some other structure for support, which displaces clots, making it difficult to capture all of the clot material.

### Disclosure of The invention

The present invention seeks to provide an improved obstruction removal assembly according to claim 1.

Preferred embodiments are described in the dependent claims. In embodiments of the present invention there is provided an obstruction removal assembly for removing an obstruction from a body vessel, including: an elongate catheter member; an expandable retrieval device including a proximal end and a distal end; the retrieval device being movable between a position within the catheter member and an extended position substantially beyond the catheter member; wherein the retrieval device is expandable when in said expanded position into a tapered shape having a chamber for receiving an obstruction the chamber being open from an access point; said tapered shape forming a smooth taper between the access point of the chamber and the proximal end of the retrieval device and being such that the proximal end of the retrieval device remains inside the catheter member; and a withdrawal mechanism coupled to the retrieval device operable to withdraw the retrieval device into the catheter member.

The withdrawal mechanism operates once the retrieval device has captured an obstruction, in the preferred embodiment a thrombus, to pull the thrombus into the catheter element. Thus, the obstruction can be captured and then held securely in the assembly for complete removal from the patient.

In the preferred embodiment, the retrieval device is expandable by unwrapping to provide a substantially conical trapping member and is rewrappable for withdrawal into the catheter element. Advantageously, the withdrawal mechanism is operable to cause said rewrapping of the retrieval device.

The catheter member may include an internal wall which provides a wrapping force to assist in rewrapping of the retrieval device.

Advantageously, the device is in the form of a film or sheet. Advantageously, the retrieval device is openable to provide an open cone and an portion which extends in a proximal direction beyond the cone, said portion providing for rewrapping to the device upon withdrawal into the catheter element. Said proximally extending portion preferably extends to either side of the device at the point of opening of the cone. The arrangement is such as to provide for the proximally extending portion to assist in the rewrapping or coiling of the device upon its withdrawal into the catheter element.

The film or sheet may be apertured or pierced. The area of the perforations or apertures may be less than 50% of the area of the film or sheet.

In the preferred embodiment, the device is in the form of a quadrilateral sheet of material when viewed flat, most preferably of kite, rhombus or diamond shape. Such a shape allows the retrieval device,
including the conical portion and the proximally extending portion to be formed integrally in terms of shape and in particular allows for the proximally extending portion to extend to the extremities of the film or sheet parts which form the open ends of the cone. In this manner, the proximally extending portion can act to provide a rewrapping force by pulling the ends of the film or sheet at the open end of the cone closed again.

In the preferred embodiment, the withdrawal mechanism includes a pulling element operable on the proximal end of the retrieval device to urge the latter into a wrapped configuration. The pulling element is advantageously a cord or wire able to be pulled in a proximal direction.

The arrangement is such as to enable the retrieval device to capture a thrombus or other obstruction and then to close in on itself by rewrapping (or recoiling), whereupon the thrombus is compressed in the retrieval device. Once rewrapped in this manner, the retrieval device can be withdrawn into the catheter element with the thrombus held therewithin. Thus, the thrombus can be removed completely in a safe manner.

Advantageously, the catheter element is an aspiration catheter. As such, the catheter element can assist in the removal of the obstruction from the patient and in particular with the absorption of the obstruction into the catheter element. Moreover, as the aspiration force of such a catheter is largely dependent upon the dimensions of the catheter, that is of its internal cross-sectional area, this arrangement can be used also with small diameter aspiration caterers as the retrieval device will assist in the retrieval of the thrombus.

Advantageously, the retrieval device is formed from a shape memory material, preferably a shape memory metal, alloy or polymer. In the preferred embodiment, the device is formed from Nitinol.

Advantageously, the catheter has an internal diameter of about 1 French (about 0.3 mm) or less.

### Brief Description of the Drawings

Embodiments of the present invention are described below, by way of example only, with reference to the accompanying drawings, in which:
Figure 1 shows a perspective view of an exemplary clot removal device in its expanded configuration;
Figure 2 shows a perspective view of the clot removal device of Figure 1 in its compressed or wrapped configuration;
Figure 3 shows a perspective view of the clot removal device of Figure 1 in a flattened configuration prior to wrapping;
Figures 4 to 8 illustrate schematically use of the clot removal device of Figure 1 to remove a clot from a blood vessel;
Figure 9 shows a perspective view of a clot removal device in accordance with the present invention, in its expanded configuration; and
Figures 12 to 13 show elevational views of the retrieval device of Figure 9 in various stages of withdrawal into a catheter.

### Detailed Description

It is to be understood that the Figures are schematic and do not show the various components to their actual scale. In many instances, the Figures show scaled up components to assist the reader in the understanding of the features taught herein.

In this description, the term distal is used to refer to an end of a component which in use is furthest from the surgeon during the medical procedure, including within a patient. The term proximal is used to refer to an end of a component closest to the surgeon and which, in practice, may be in or adjacent an external manipulation part of the deployment or treatment apparatus.

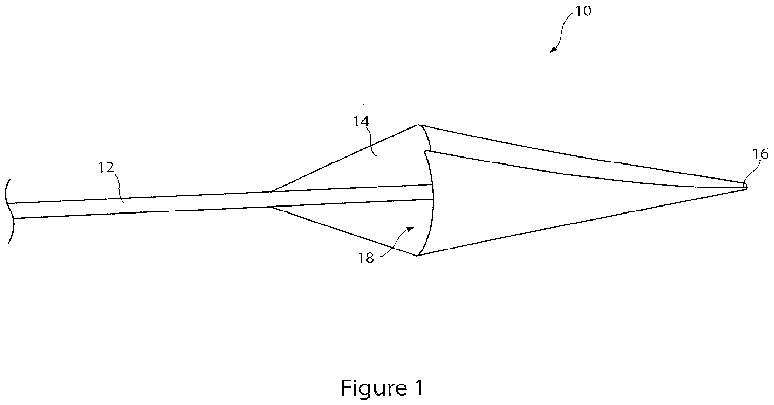

Figure 1 illustrates an exemplary obstruction removal assembly 10. The removal assembly 10 includes a wire 12 that may be in the form of a guide wire or a similar structure, or which may be of a more substantial rod-like structure, still flexible to be able to pass through a patient's vasculature. At the distal end of the wire 12 is a retrieval device 14 which in this embodiment has a conical form. The retrieval device 14 has an apex 16 at the distal end of the retrieval assembly 10. The base of the forms an opening 18 into the retrieval device 14. The opening 18 is located proximally of the apex 16.

Preferably, the retrieval device 14 is formed from a Nitinol film. Alternatively, any shape memory material, for example an alloy or polymer may be used. The Nitinol film is pre-treated in a known manner so as to be compressible but to revert to a substantially conical expanded configuration at body temperature via a shape memory mechanism, thereby forming the retrieval cone 14 in vivo.

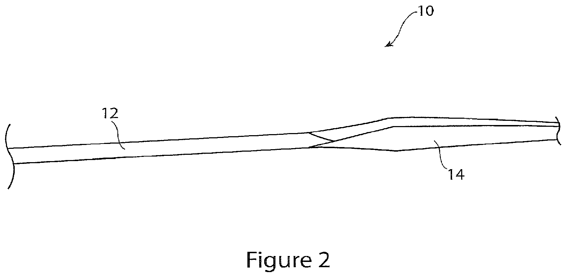

As illustrated in Figure 1, it can be seen that the walls of the retrieval cone 14 are able to overlap one another and so the cone 14 can be wrapped tightly around itself and around the wire 12 into a compressed configuration (see Figure 2). When the Nitinol film is below its Austenite finish temperature (for example, at room temperature), the Nitinol is in its martensite form and retains the compressed configuration ready for delivery (see below). Since the Nitinol film is very thin, this arrangement allows a very small compressed configuration to be achieved.

For very small lumen applications, for instance for cerebral applications, the retrieval cone 14 may have a closed diameter able to fit within a micro catheter having an internal diameter of 1 French (0.33 mm) or less. In its expanded configuration (as shown in Figure 1) the diameter of the retrieval cone 14 at the position of the opening 18 (that is at the base of the cone) may be 1 to 10 mm, for example.

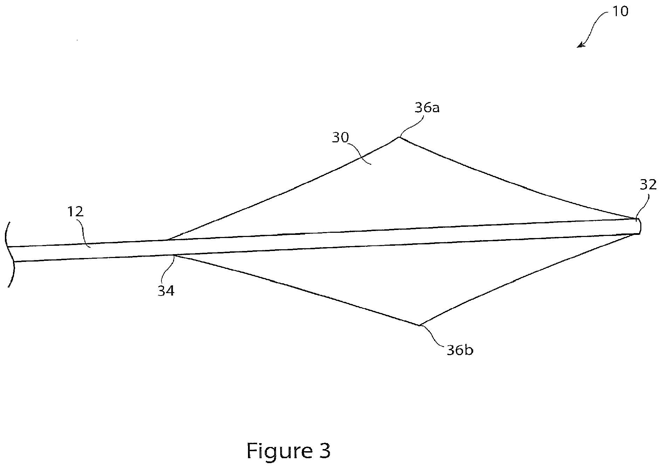

The retrieval cone 14 is preferably formed from a substantially kite, diamond of rhombus shaped sheet of Nitinol film 30, as illustrated in Figure 3 (hereinafter referred to as kite shaped). The Nitinol film preferably has a length (between the distal end 32, which forms the apex 16 of the retrieval cone and the proximal 34 of the film 30) of 12 mm. The spacing between points 36a and 36b is, in this embodiment, at least 4-6 mm up to around 30 mm or more.

The film preferably has a thickness of 0.1 to 0.5 millimetres.

The Nitinol film is fixed to the wire 12 in any suitable manner, for example, by laser, soldering, crimping, gluing or tying. The skilled person will appreciate that other methods may also be used. The kite-shaped sheet of Nitinol film 30 is formed into a cone as illustrated in Figure 1 by overlapping the points 36a and 36b. The Nitinol film 30 is treated in a known manner to provide shape memory so that, at body temperature, the Nitinol film 30 is able to form the retrieval cone 14 as described below.

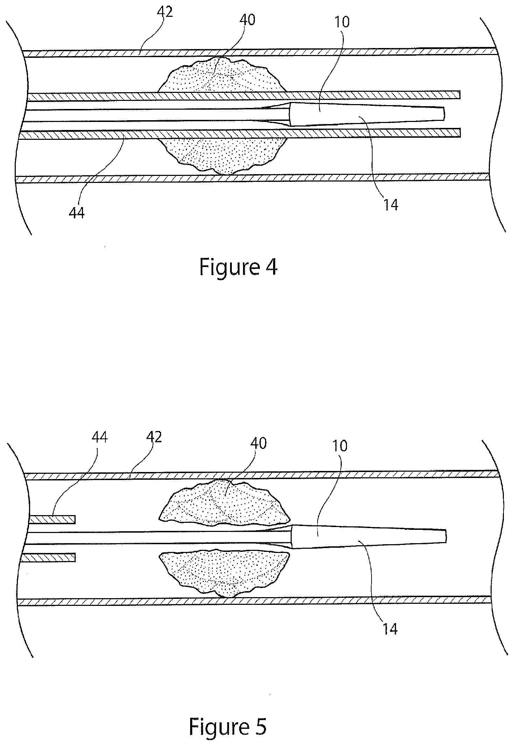

Figures 4 to 8 illustrate use of the retrieval device 10 in the removal of a blood clot 40 from a blood vessel 42. As shown in Figure 4, the device 10 is held within a micro catheter 44 with the retrieval cone 14 in a compressed configuration. The micro catheter preferably has a diameter of less than 1 French (0.33 mm), and so is able to be passed through a thrombus 40 without disturbing the thrombus 40 or otherwise causing the thrombus 40 to break up.

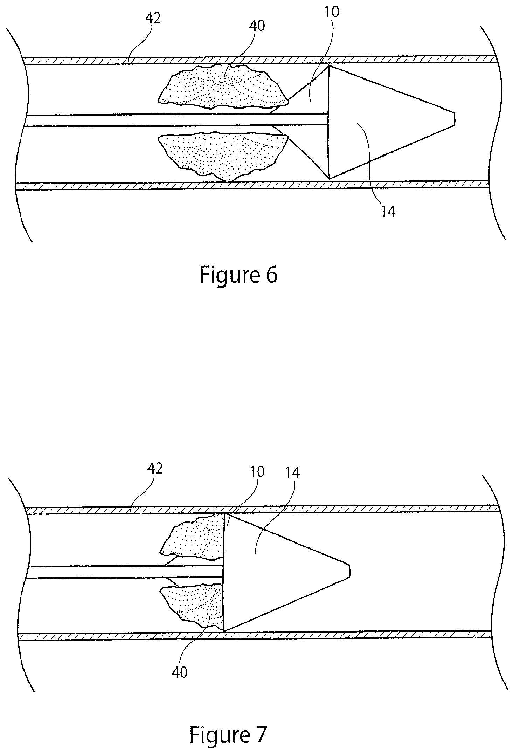

Once the clot removal device 10 has been advanced sufficiently that the proximal end of the retrieval cone 14 is beyond the thrombus 40, the micro catheter 44 is withdrawn (see Figure 5). The retrieval cone 14,
released from the confines of the micro catheter 44, is then able to expand into its shape memory conical form (see Figure 6). As can be seen, it is preferable that the device 14 is sized such that the open end of the cone extends to the walls of the lumen 42 to be treated.

It will be appreciated that in some instances and for the removal of hard debris such as plaque the device will not necessarily penetrate the
thrombus or other obstruction and instead may be fed beyond this to one side of the obstruction or thrombus.

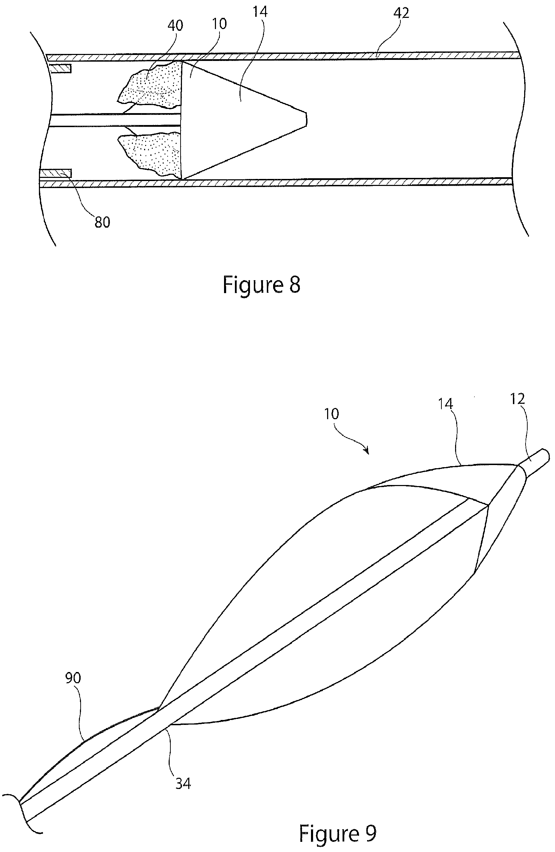

Once the retrieval cone 14 is in its expanded configuration, the device 10 can be pulled back in a proximal direction, as shown in Figure 7. As this is done, the thrombus 40 is caught within the retrieval cone 14. As the clot retrieval device 10 is further withdrawn proximally, the blood clot 40 is withdrawn therewith (see Figure 8).

In some examples of the method (and as illustrated in Figure 8), an aspiration catheter 80 may be introduced into the blood vessel 42 and may be used to assist in removal of the clot 40 from the retrieval cone 14. However, where the blood clot 40 is located in a very narrow vessel, it may be difficult to introduce an aspiration catheter 80, in which case, the blood clot 40 can simply be removed during withdrawal of the clot removal device 10 from the patient. In other words, the clot removal device 10 enables a clot (thrombus) 40 to be removed from a blood vessel 42 without the need for aspiration.

Figure 9 illustrates an embodiment of obstruction retrieval device 10 in accordance with the present invention. As with the example of Figure 1, the retrieval device 10 includes a wire or rod 12, which may be of guide wire type, for example. A retrieval cone 14, again preferably formed from a Nitinol film, is attached at its tip or apex to the distal end of the wire 12. The retrieval cone 14 is provided with a withdrawal member 90, which may be a wire, cord or similar structure. The withdrawal member 90 is, in this embodiment, attached to the proximal end 34 of the Nitinol film in any suitable manner, such as by suturing, soldering, crimping or gluing. For this purpose, the proximal end of the device 14 is not fixed to the wire or rod 12 but can slide therealong. In practice, the user can pull the withdrawal member 90 in the proximal direction to cause the retrieval cone 14 to close by rewrapping.

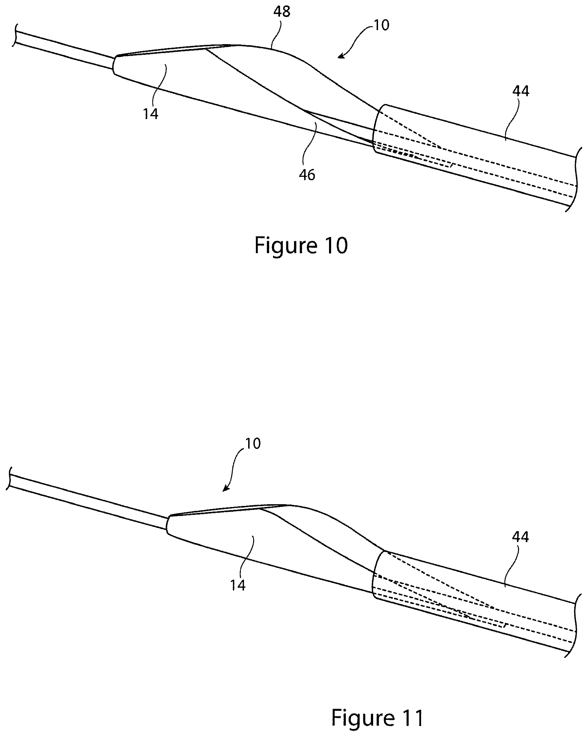

Figures 10 to 13 show how the embodiment of Figure 9 operates in practice.

Referring first to Figure 10, the device 10 is shown in what could be termed its extended state, in which the device 10 extends beyond the catheter 44 and the device 10 is opened to form the cone 14, which tapers towards the distal end of the assembly 10. In the configuration of Figure 10, the cone 14 is in a state for catching a thrombus or other obstruction in a body vessel. Typically, the cone 14 at its widest extent is at least as wide as the lumen into which is it intended to be deployed. In practice, the end 48 of the cone 14 may be designed to be wider than the lumen so as to ensure complete spanning of the lumen during the thrombus/obstruction capture operation.

It will be seen in Figure 10 that the shape of the device 10, that is its kite, diamond or rhombus shape, provides the device 10 with a proximal end 46 which tapers downwardly in the proximal direction of the assembly 10 and at its end into the catheter 44, doing so while presenting a smooth transition in material from the widest portion 48 to the end 34 at the wire or cord 90. This opposing taper assists in the subsequent collapse or rewrap of the device 10 during the thrombus retrieval operation, as described below.

The proximal end 34 of the device 10 remains, in this embodiment, within the catheter 44, retaining a curved radial shape to the proximal end of the device 10, assisting in its subsequent withdrawal into the catheter 44.

Once a thrombus or other obstruction has been caught in the cone 14, the device 10 can be withdrawn into the catheter 44. By pulling on the wire or cord 90, typically in conjunction with pulling into the catheter 44, the device is caused to rewrap on itself. During this process, the thrombus or other
obstruction is wrapped into the film forming the device 10 and held therewithin.

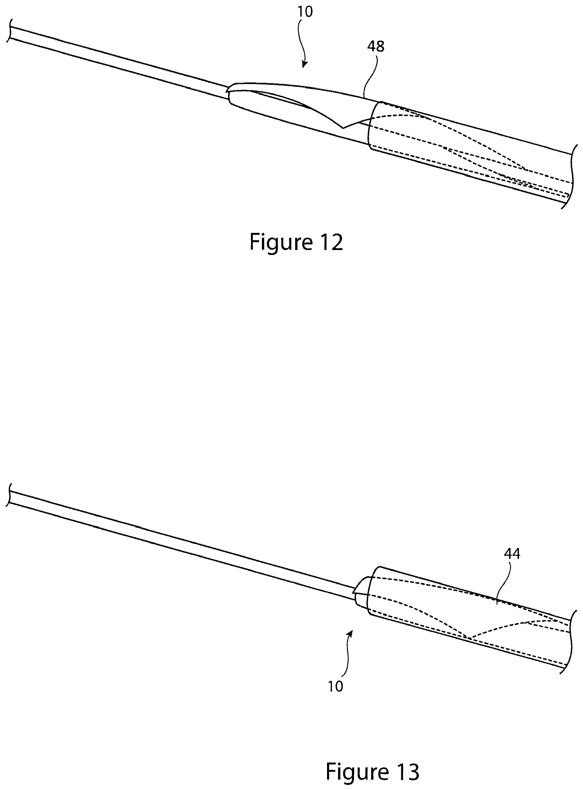

Figures 12 and 13 show the device 10 as it is progressively increasingly pulled into the catheter 44 and as it is progressively wrapped tighter. In its final stage, the device 10 is completely wrapped and held within the catheter 44, with the thrombus held therewithin and thus safely captured for removal from within the patient.

In some embodiments, it might be advantageous to use the catheter 44 as an aspiration device, that is to apply aspiration as the device 10 is being pulled to its distal end so as to assist in the retrieval of a thrombus. In other embodiments, there may be provided a second catheter, typically of significantly larger diameter, as an aspiration catheter.

Thus, a surgeon or clinician uses the withdrawal member 90 to pull the retrieval cone 14 in a proximal direction over the wire 12. The configuration of the cone, with its smooth transition or taper between its widest point at the opening of the capture chamber and its proximal end allows the retrieval cone 14 to be compressed, rewrapped or coiled as it is withdrawn into the catheter 44. The retrieval cone 14, the clot 40 and the catheter 44 can then together be withdrawn from the patient.

Although the devices described herein has been described particularly in relation to the removal of blood clots (thrombi), the skilled person will appreciate that the principles disclosed can equally be used in the removal of other types of obstruction from other types of body vessel, such as plaque or other debris.

What have been described and illustrated herein are preferred embodiments of the invention along with some of its variations. The terms, descriptions and Figures used herein are set forth by way of illustration only and are not meant as limitations. Those skilled in the art will recognize that many variations are possible within the scope of the claims, in which all terms are meant in their broadest reasonable sense unless otherwise indicated.
For example, whilst the illustrated embodiments use a non-perforated Nitinol film, the skilled person will appreciate that, in some embodiments, it may be advantageous to form the retrieval cone 14 from a perforated Nitinol film.

The features of the various embodiments described above and their modifications may be substituted for or combined with one another as desired. It is also to be understood that the various features of the dependent claims appended hereto may be used with one another in any desired combination of those claims.

## Claims

1. An obstruction removal assembly (10) for removing an obstruction (40) from a body vessel, including:
an elongate catheter member (44);
an expandable retrieval device (14) including a proximal end (34) and a distal end (32); the retrieval device being movable between a position within the catheter member and an extended position substantially beyond the catheter member; wherein the retrieval device is expandable when in said expanded position into a tapered shape having a chamber for receiving an obstruction, the chamber being open from an access point; said tapered shape forming a smooth taper between the access point of the chamber and the proximal end of the retrieval device; and a withdrawal mechanism (90) coupled to the retrieval device operable to withdraw the retrieval device into the catheter member;
**characterised in that** the retrieval device (14) is in the form of a film or sheet which is of quadrilateral shape when viewed flat, the assembly being configured such that with the proximal end (34) of the expandable retrieval device (14) remaining inside the catheter member (44) the proximal end (34) retains a curved radial shape thereby facilitating wrapping of the device (14) as it is withdrawn into the catheter member (44).

2. An assembly (10) according to claim 1, wherein the withdrawal mechanism (90) includes a pulling element for pulling the retrieval device (14) into the catheter member (44) and thereby pulling an obstruction captured in the retrieval device (14) into the catheter element (44).

3. An assembly (10) according to claim 1 or 2, wherein the retrieval device (14) is expandable by unwrapping to provide a substantially conical trapping member and is rewrappable for withdrawal into the catheter member (44).

4. An assembly (10) according to claim 3, wherein the withdrawal mechanism (90) is operable to cause said rewrapping of the retrieval device (14).

5. An assembly (10) according to claim 3 or 4, wherein said catheter member (44) includes an internal wall which provides a wrapping force to assist in rewrapping of the retrieval device (14).

6. An assembly (10) according to any preceding claim, wherein the film or sheet of the retrieval device (14) is openable to provide an open cone and a portion (46) which extends in a proximal direction beyond the cone, said portion providing for rewrapping of the device upon withdrawal into the catheter member (44).

7. An assembly (10) according to claim 6, wherein the said proximally extending portion (46) extends to either side of the retrieval device (14) at the point of opening of the cone.

8. An assembly (10) according to any preceding claim, wherein the film or sheet is perforated, apertured or pierced, and wherein the area of the perforations or apertures is less than 50 % of the area of the film or sheet.

9. An assembly (10) according to any preceding claim, wherein the film or sheet is in the form of one of: a kite, a rhombus and a diamond shape.

10. An assembly (10) according to any preceding claim, wherein the withdrawal mechanism (90) includes a pulling element able to pull on the proximal end of the retrieval device to urge the latter into a wrapped configuration.

11. An assembly according to claim 10, wherein the pulling element is a cord or wire able to be pulled in a proximal direction.

12. An assembly (10) according to any preceding claim, wherein the catheter member (44) is an aspiration catheter.

13. An assembly (10) according to any preceding claim, wherein the retrieval device (14) is formed from a shape memory material.

14. An assembly (10) according to claim 13, wherein the retrieval device (14) is formed from one of: a shape memory metal, alloy or polymer.

## Patentansprüche

1. Obstruktionsentfernungsanordnung (10) zur Beseitigung einer Obstruktion (40) aus einem Körpergefäß, umfassend:
ein langgestrecktes Katheterelement (44);
eine expandierbare Rückholvorrichtung (14) mit einem proximalen Ende (34) und einem distalen Ende (32); wobei die Rückholvorrichtung zwischen einer Position innerhalb des Katheterelements und einer ausgezogenen Position wesentlich über das Katheterelement hinaus bewegbar ist; wobei die Rückholvorrichtung in der expandierten Position in eine verjüngte Form mit einer Kammer zur Aufnahme einer Obstruktion expandierbar ist, wobei die Kammer von einem Zugangspunkt offen ist; wobei die verjüngte Form eine glatte Verjüngung zwischen dem Zugangspunkt der Kammer und dem proximalen Ende der Rückholvorrichtung bildet; und einem Rückzugsmechanismus (90), der mit der Rückholvorrichtung verbunden ist und zum Zurückziehen der Rückholvorrichtung in das Katheterelement betätigbar ist;
**dadurch gekennzeichnet, dass** die Rückholvorrichtung (14) in Form einer Folie oder eines Flächenstücks vorliegt, die bzw. das flach gesehen vierseitig geformt ist, wobei die Anordnung derart ausgelegt ist, dass, während das proximale Ende (34) der expandierbaren Rückholvorrichtung (14) im Katheterelement (44) bleibt, das proximale Ende (34) eine gebogene radiale Gestalt behält, wodurch Aufwickeln der Vorrichtung (14) beim Zurückziehen in das Katheterelement (44) erleichtert wird.

2. Anordnung (10) nach Anspruch 1, wobei der Rückzugsmechanismus (90) ein Ziehelement zum Ziehen der Rückholvorrichtung (14) in das Katheterelement (44) aufweist, wobei eine in der Rückholvorrichtung (14) festgehaltene Obstruktion in das Katheterelement (44) gezogen wird.

3. Anordnung (10) nach Anspruch 1 oder 2, wobei die Rückholvorrichtung (14) durch Abwickeln expandierbar ist, um ein im Wesentlichen konisches Einfangelement bereitzustellen, und zum Zurückziehen in das Katheterelement (44) wieder aufgewickelt werden kann.

4. Anordnung (10) nach Anspruch 3, wobei der Rückzugsmechanismus (90) betätigbar ist, derart, dass er verursacht, dass die Rückholvorrichtung (14) wieder aufgewickelt wird.

5. Anordnung (10) nach Anspruch 3 oder 4, wobei das Katheterelement (44) eine Innenwand aufweist, die eine Aufwickelkraft zur Unterstützung des Wiederaufwickelns der Rückholvorrichtung (14) bereitstellt.

6. Anordnung (10) nach einem der vorhergehenden Ansprüche, wobei die Folie oder das Flächenstück der Rückholvorrichtung (14) geöffnet werden kann, um einen offenen Kegel und einen Abschnitt (46) bereitzustellen, der sich in eine proximale Richtung über den Kegel hinaus erstreckt, wobei der Abschnitt für das Wiederaufwickeln der Vorrichtung beim Zurückziehen in das Katheterelement (44) sorgt.

7. Anordnung (10) nach Anspruch 6, wobei der sich proximal erstreckende Abschnitt (46) sich zu beiden Seiten der Rückholvorrichtung (14) an dem Punkt der Öffnung des Kegels erstreckt.

8. Anordnung (10) nach einem der vorhergehenden Ansprüche, wobei die Folie oder das Flächenstück perforiert, mit Öffnungen versehen oder durchstochen ist, und wobei die Fläche der Perforationen oder Öffnungen weniger als 50% der Fläche der Folie oder des Flächenstücks beträgt.

9. Anordnung (10) nach einem der vorhergehenden Ansprüche, wobei die Folie oder das Flächenstück in Form eines von: einem Drachen, einem Rhombus und einer Diamantgestalt vorliegt.

10. Anordnung (10) nach einem der vorhergehenden Ansprüche, wobei der Rückzugsmechanismus (90) ein Ziehelement aufweist, das am proximalen Ende der Rückholvorrichtung ziehen kann, um letztere in eine aufgewickelte Konfiguration zu drängen.

11. Anordnung nach Anspruch 10, wobei das Ziehelement eine Schnur oder ein Draht ist, die bzw. der in eine proximale Richtung gezogen werden kann.

12. Anordnung (10) nach einem der vorhergehenden Ansprüche, wobei das Katheterelement (44) ein Aspirationskatheter ist.

13. Anordnung (10) nach einem der vorhergehenden Ansprüche, wobei die Rückholvorrichtung (14) aus einem Formgedächtnismaterial besteht.

14. Anordnung (10) nach Anspruch 13, wobei die Rückholvorrichtung (14) aus einem von: Formgedächtnismetall, -legierung oder -polymer, besteht.

## Revendications

1. Ensemble (10) d'élimination d'obstruction destiné à éliminer une obstruction (40) d'un vaisseau corporel, comprenant :
un élément (44) formant cathéter allongé ;
un dispositif (14) de récupération extensible comprenant une extrémité (34) proximale et une extrémité (32) distale ; le dispositif de récupération étant mobile entre une position à l'intérieur de l'élément formant cathéter et une position étendue sensiblement au-delà de l'élément formant cathéter ; dans lequel le dispositif de récupération est extensible lorsqu'il est dans ladite position étendue en une forme fuselé présentant une chambre destinée à recevoir une obstruction, la chambre étant ouverte depuis un point d'accès ; ladite forme fuselée formant un fuseau lisse entre le point d'accès de la chambre et l'extrémité proximale du dispositif de récupération ; et un mécanisme (90) de retrait accouplé au dispositif de récupération servant au retrait du dispositif de récupération à l'intérieur de l'élément formant cathéter ;
**caractérisé en ce que** le dispositif (14) de récupération se présente sous la forme d'un film ou d'une feuille ayant une forme quadrilatérale lorsqu'ils sont visualisés à plat, l'ensemble étant configuré de telle sorte qu'avec l'extrémité (34) proximale du dispositif (14) de récupération extensible restant à l'intérieur de l'élément (44) formant cathéter, l'extrémité (34) proximale retient une forme radiale courbe, facilitant ainsi l'enroulement du dispositif (14) au fur et à mesure de son retrait dans l'élément (44) formant cathéter.

2. Ensemble (10) selon la revendication 1, dans lequel le mécanisme (90) de retrait comprend un élément de traction destiné à tirer le dispositif (14) de récupération dans l'élément (44) formant cathéter et à tirer ainsi une obstruction saisie dans le dispositif (14) de récupération dans l'élément (44) formant cathéter.

3. Ensemble (10) selon la revendication 1 ou 2, dans lequel le dispositif (14) de récupération est extensible par déroulement pour fournir un élément de piégeage sensiblement conique et peut être réenroulé pour son retrait dans l'élément (44) formant cathéter.

4. Ensemble (10) selon la revendication 3, dans lequel le mécanisme (90) de retrait pouvant servir à entraîner ledit réenroulement du dispositif (14) de récupération.

5. Ensemble (10) selon la revendication 3 ou 4, dans lequel ledit élément (44) formant cathéter comprend une paroi interne qui fournit une force d'enroulement pour aider au réenroulement du dispositif (14) de récupération.

6. Ensemble (10) selon l'une quelconque des revendications précédentes, dans lequel le film ou la feuille du dispositif (14) de récupération peuvent être ouverts pour fournir un cône ouvert et une partie (46) qui s'étend dans une direction proximale au-delà du cône, ladite partie fournissant le réenroulement du dispositif au moment du retrait dans l'élément (44) formant cathéter.

7. Ensemble (10) selon la revendication 6, dans lequel ladite partie (46) s'étendant proximalement s'étend de chaque côté du dispositif (14) de récupération au niveau du point d'ouverture du cône.

8. Ensemble (10) selon l'une quelconque des revendications précédentes, dans lequel le film ou la feuille sont perforés, ouverts ou percés, et dans lequel la zone des perforations ou des ouvertures est inférieure à 50 % de la zone du film ou de la feuille.

9. Ensemble (10) selon l'une quelconque des revendications précédentes, dans lequel le film ou la feuille se présentent sous l'une des formes suivante : un cerf-volant, un losange et une forme de diamant.

10. Ensemble (10) selon l'une quelconque des revendications précédentes, dans lequel le mécanisme (90) de retrait comprend un élément de traction capable de tirer sur l'extrémité proximale du dispositif de récupération pour forcer ce dernier dans une configuration enroulée.

11. Ensemble selon la revendication 10, dans lequel l'élément de traction est un cordon ou un fil métallique capable d'être tiré dans une direction proximale.

12. Ensemble (10) selon l'une quelconque des revendications précédentes, dans lequel l'élément (44) formant cathéter est un cathéter d'aspiration.

13. Ensemble (10) selon l'une quelconque des revendications précédentes, dans lequel le dispositif (14) de récupération est constitué d'un matériau à mémoire de forme.

14. Ensemble (10) selon la revendication 13, dans lequel le dispositif (14) de récupération est constitué d'un des matériaux suivants : un métal à mémoire de forme, un alliage ou un polymère.
